(19) Europäisches Patentamt
European Patent Office
Office européen des brevets

(11) Publication number: **0 490 878 A1**

# (12) EUROPEAN PATENT APPLICATION

(21) Application number: **92102929.4**

(22) Date of filing: **06.03.90**

(51) Int. Cl.[5]: **C07H 15/252**, C07H 19/02, A61K 31/70

This application was filed on 21 - 02 - 1992 as a divisional application to the application mentioned under INID code 60.

(30) Priority: **13.03.89 GB 8905668**

(43) Date of publication of application: **17.06.92 Bulletin 92/25**

(60) Publication number of the earlier application in accordance with Art.76 EPC: **0 387 661**

(84) Designated Contracting States: **AT BE CH DE DK ES FR GB GR IT LI NL SE**

(71) Applicant: **FARMITALIA CARLO ERBA S.r.L.**
**Via Carlo Imbonati 24**
**I-20159 Milano(IT)**

(72) Inventor: **Suarato, Antonino**
**Via Degli Imbriani 39**
**I-20138 Milan(IT)**
Inventor: **Angelucci, Francesco**
**Via Washington 106**
**I-20100 Milan(IT)**
Inventor: **Bargiotti, Alberto**
**Via Donati 8**
**I-20100 Milan(IT)**
Inventor: **Grandi, Maria**
**Piazza 5 Giornate 6**
**I-20100 Milan(IT)**
Inventor: **Pezzoni, Gabriella**
**Via Pomezia 10/a**
**I-20100 Milan(IT)**

(74) Representative: **Giambrocono, Alfonso, Dr. Ing. et al**
**Ing. A. Giambrocono & C. S.r.l. Via Rosolino Pilo 19/B**
**I-20129 Milano(IT)**

(54) **New 3'-(4-Morpholinyl)- anthracycline derivatives.**

(57) Anthracycline glycosides of general formula I:

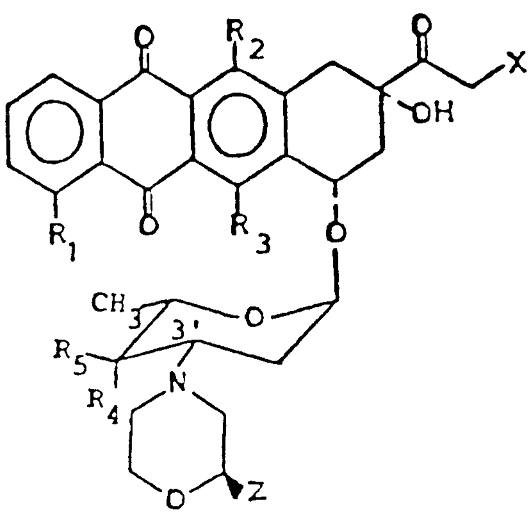

wherein Z is hydrogen

EP 0 490 878 A1

X represents hydrogen or hydroxy;
$R_1$ amino;
$R_2$ and $R_3$ both represent hydroxy $R_5$ represents hydrogen and $R_4$ is hydroxy;
and pharmaceutically acceptable salts thereof.

The invention relates to anthracycline glycosides, to processes for their preparation and to pharmaceutical compositions containing them.

The invention provides anthracycline glycosides having the general formula I:

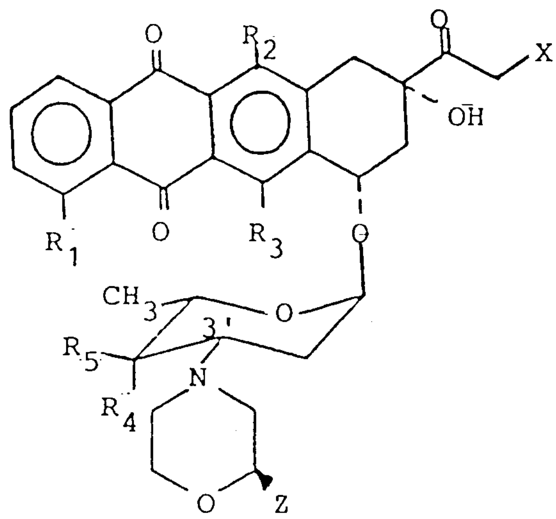

wherein Z is hydrogen

X represents hydrogen or hydroxy;

$R_1$ represents amino;

$R_2$ and $R_3$ both represent hydroxy; and

$R_5$ is hydrogen and $R_4$ is hydroxy;

and pharmaceutically acceptable salts thereof

b) : 4-demethoxy-4-amino-3'-deamino-3'-(4-morpholinyl)-daunorubicin

($X = H$, $R_1 = NH_2$, $R_2 = R_3 = R_4 = OH$, $R_5 = H$, $Z = H$)

The new anthracycline glycoside antibiotics of the invention are prepared by the formation of a morpholinyl ring at C-3' on the sugar moiety of the antitumor anthracycline glycosides of general formula II:

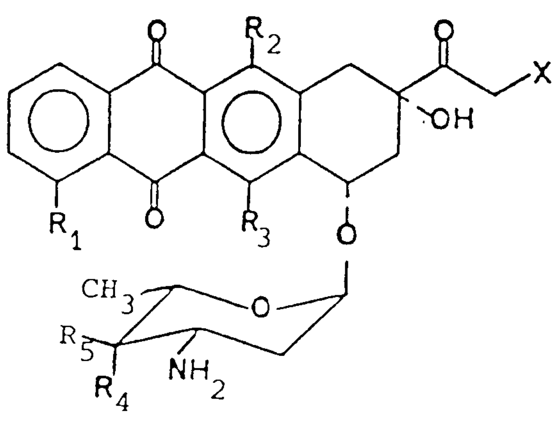

wherein $R_1$, $R_2$, $R_3$, $R_4$, $R_5$ and X are as above defined or a salt thereof, for example a pharmaceutically acceptable acid addition salt such as the hydrochloride salt. An anthracycline glycoside of formula I wherein $Z = H$ or a pharmaceutically acceptable salt is therefore prepared by reacting an anthracycline glcoside of formula II or a salt thereof, for example a pharmaceutically acceptable acid addition salt such as the hydrochloride salt, with bis(2-iodoethyl)ether and, if desired, convering the anthracycline glycoside of formula I wherein Z is H thus obtained into a pharmaceutically acceptable salt thereof.

More particularly the reaction for preparing 3'-(4-morpholino) anthracycline derivatives of formula I is typically carried out using an excess of bis(2-iodoethyl)ether in an anhydrous polar organic solvent, such as dimethylformamide. The reaction is generally carried out at room temperature. It can be usually completed in one day. The desired product is isolated from the reaction mixture, for example by solvent extraction. It

may be purified by chromatography, for example column chromatography. It may be transformed into the hydrochloride salt, for example by treatment with methanolic hydrogen chloride.

In one embodiment, therefore, the anthracycline glycoside of formula II or salt thereof dissolved in an anhydrous polar solvent is reacted, at room temperature and for 24 hours, with an excess of bis(2-iodoethyl)ether in the presence of triethylamine; the pH of the reaction mixture, previously diluted with water, is adjusted to 7.5 using an aqueous solution of sodium hydrogen carbonate; the reaction mixture is extracted with methylene chloride; the organic solvent is removed under reduced pressure; and, after purification of the crude product on a column of silica gel using a mixture of methylene chloride and methanol (98:2 v/v) as eluting agent, the product is isolated as the hydrochloride salt by addition of a methanolic solution of anhydrous hydrogen chloride.

The starting materials for making the new 3'-(4-morpholino) glycosides of the invention is:

- the well known 4-demethoxy-4-amino-daunorubicin (C6: $X=H$, $R_1=NH_2$, $R_5=H$, $R_2=R_3=R_4=OH$) and salt thereof such as the hydrochloride salt.

The invention provides pharmaceutical compositions comprising an anthracycline glycoside of formula I or a pharmaceutically acceptable salt thereof, together with a pharmaceutically acceptable diluent or carrier. Coventional carriers and diluents may be used. The composition may be formulated and administered in conventional manner.

The compounds of the invention are useful in methods Of treatment of the human or animal body by therapy. They are useful as antitumor agents. A therapeutically effective amount is administered to a patient. An amount sufficient to inhibit the growth of the tumour may be administered. The tumor may be a Colon adenocarcinoma or Gross leukaemia tumor.

The biological activity of the compounds according to the invention was tested in vitro against LoVo (human colon adenocarcinoma) and LoVo/DX cells in comparison with doxorubicin. The results are shown in Table 1. The compounds of the invention were also tested in vivo against P388 and P388/DX murine leukemia in comparison with doxorubicin. The results are shown in Table 2. All compounds were tested as their hydrochlorides.

Table 1 - *in vitro* activity

| Compounds | Cytotoxicity after 4h of treatment (IC50* = ng/ml) | | |
|---|---|---|---|
| | LoVo | LoVo/DX | R.I.** |
| Doxorubicin | 60 | 2180 | 36 |
| Ib | 13.7 | 28.5 | 2.1 |

* IC50 = concentration inhibiting by 50% colony growth

** R.I. = Resistance Index = (IC50 LoVo/DX)/(IC50 LoVo)

Table 2 - Antileukemic activity against P388 sensitive and resistant to doxorubicin

| Compounds | P388 ( iv, iv +1) O.D.* (mg/Kg) | %T/C** | P388/DX Johnson O.D.* (mg/Kg) | (iv , iv +1) %T/C** |
|---|---|---|---|---|
| Doxorubicin | 13 | 250 | 13 | 100 |
| Ib | 0.76 | 156 | 0.575 | 167 |

iv means that tumor has been implanted intravenously;

iv+1 means that treatment begins one day after said implantation.

* Optimal dose: maximum tolerated dose (LD10);

** Median survival time of treated mice/median survival time of controls x 100;

*** 3 of 10 mice were considered long term survivors at the end of the experiments (90 days).

The following Examples illustrate the invention.

Thin layer chromatography (TLC) measurements were made on Kieselgel plates (Merck $F_{254}$) using methylene chloride/ethanol (95/5 by volume) as eluent. "Merck" is a Trade Mark.

Example 1

4-demethyl-6-deoxy-3'-deamino-3'-(4-morpholinyl)doxorubicin (a)

To a solution of 0.15g (0.27 mmole) of 4-demethyl-6-deoxy-doxorubicin hydrochloride (C5) in 5 ml of anhydrous dimethylformamide, was added 1g (3.07 mmole) of bis(2-iodoethyl)ether and 0.075 ml (0.535 mmole) of triethylamine. The reaction mixture was stirred at room temperature for one day, then the mixture was diluted with 100 ml of water and the pH was adjusted to 7.5 with a solution of aqueous sodium hydrogen carbonate. The aqueous layer was extracted with methylene chloride three times. The organic phase was dried over anhydrous sodium sulphate and the solvent removed under reduced pressure. The residue was chromatographed on a column of silica gel using methylene chloride/methanol (98:2 v/v) as eluting system to give 70 mg (yield 42%) of the title compound (a) which was isolated as hydrochloride salt after addition with a methanolic solution of anhydrous hydrogen chloride.

m.p. 161-162° C (dec.); Rf:0.19; FD-MS: m/z 583 (M+.);

$^1$HNMR (200 MHz, DMSO) inter alia δ:

12.94 (s, 1H, 11-OH,), 12.53 (s, 1H, 4-OH), 7.9-7.7 (m, 3H, 6-H, 1-H, 2-H), 5.77 (d, J=5.7 Mz, 1H, 4'-OH), 5.42 (d , J=5.9, 7.4Hz, 1H, 7-H), 4.54 (s, 2H, $CH_2$ OH), 4.0-2.7 (m,

Example 2

4-demethoxy-4-amino-3'-deamino-3'-(4-morpholinyl)daunorubicin (b)

Following the procedure described in Example 1, the title compound (b) was obtained starting from 4-demethoxy-4-amino-daunorubicin (C6).
Yield 80%, m.p. 167-168 ° C (dec.); Rf:0.33; FD-MS: m/z 582 (M + .) $^1$HNMR (200 MHz, $CDCl_3$) inter alia δ: 13.73 (s, 1H, 6-OH), 13.40 (s, 1H, 11-OH), 7.67 (d, J = 6.4Hz, 1H, 1-H), 7.49 (dd, J = 6.4, 8.3Hz, 1H, 2-H), 6.97 (d, J = 8.3Hz, 3-H), 6.81 (broad, 2H, $NH_2$), 5.56 (d, J = 3.2Hz, 1H, 1'-H), 5.26 (dd, J = 2.2, 3.8Hz, 1H, 7-H), 3.68 (m, 4H, $CH_2$ $OCH_2$), 2.6-2.3 (m, 6H, $CH_2$ $NCH_2$), 2.42 (s, 3H, $COCH_3$), 1.38 (d, J = 6.6Hz, 3H, 5'-$CH_3$).

**Claims**

1. An anthracycline glycoside of general formula I:

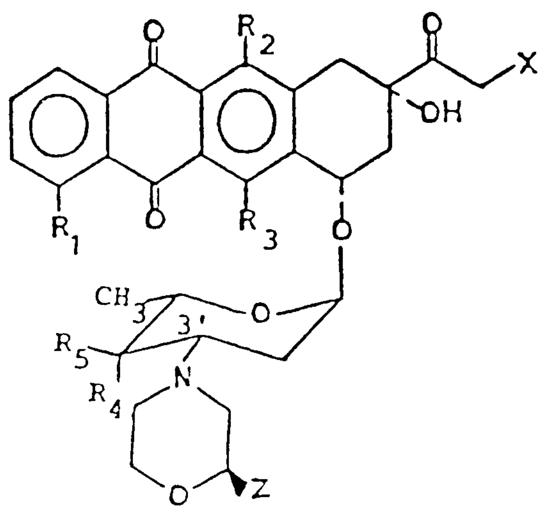

wherein Z is hydrogen;
X represents hydrogen or hydroxy;
$R_1$ represents amino;
$R_2$ and $R_3$ both represent hydroxy; and
$R_5$ is hydrogen and $R_4$ is hydroxy;
and pharmaceutically acceptable salts thereof;

2. A compound according to Claim 1, which is 4-demethoxy-4-amino-3'-deamino-3'-(4-morpholinyl)-daunorubicin

3. A process for the preparation of an anthracycline glycoside of formula I according to Claim 1 wherein Z = H, or a pharmaceutically acceptable salt thereof, which process comprises reacting an anthracycline glycoside of general formula II:

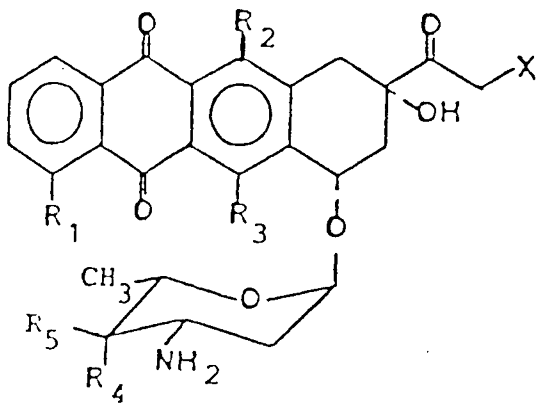

wherein $R_1$, $R_2$, $R_3$, $R_4$, $R_5$ and X are as defined in Claim 1, or a salt thereof, with bis(2-iodoethyl)ether and, if desired, converting the anthracycline glycoside of formula I wherein Z is H thus obtained into a pharmaceutically acceptable salt thereof.

**4.** A process according to claim 3 wherein the anthracycline glycoside of formula II or salt thereof dissolved in an anhydrous polar solvent is reacted, at room temperature and for 24 hours, with an excess of bis(2-iodoethyl)ether in the presence of triethylamine; the pH of the reaction mixture, previously diluted with water, is adjusted to 7.5 using an aqueous solution of sodium hydrogen carbonate; the reaction mixture is extracted with methylene chloride; the organic solvent is removed under reduced pressure; and, after purification of the crude product on a column of silica gel using a mixture of methylene chloride and methanol (98:2 v/v) as eluting agent, the product is isolated as the hydrochloride salt by addition of a methanolic solution of anhydrous hydrogen chloride.

**5.** A pharmaceutical composition comprising an anthracycline glycoside of formula I as defined in Claim 1, or a pharmaceutically acceptable acid addition salt thereof, together with a pharmaceutically acceptable carrier or diluent.

**6.** An anthracycline glycoside of formula I according to Claim 1, or a pharmaceutically acceptable salt thereof, for use as an anti-tumor agent.

**Claims for the following Contracting States : ES, GR**

**1.** A process for the preparation of an anthracycline glycoside of formula I

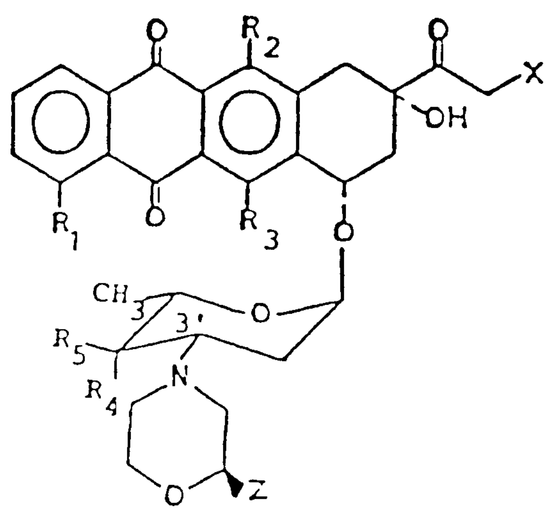

wherein Z = H,
X represents hydrogen or hydroxy;
$R_1$ represents amino;
$R_2$ and $R_3$ both represent hydroxy; and $R_5$ is hydrogen and $R_4$ is hydroxy;
and pharmaceutically acceptable salts thereof;
which process comprises reacting an anthracycline glycoside of general formula II:

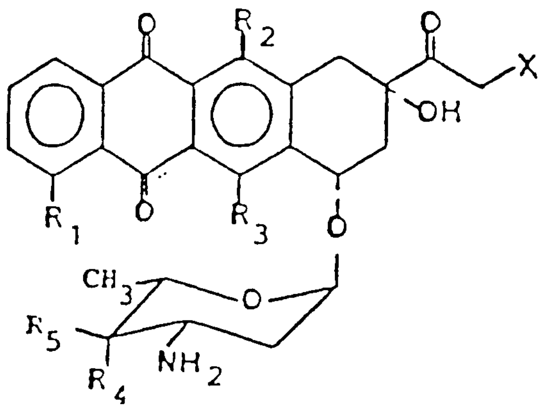

wherein $R_1$, $R_2$, $R_3$, $R_4$, $R_5$ and X are as defined above, or a salt thereof, with bis(2-iodoethyl)ether and, if desired, converting the anthracycline glycoside of formula I wherein Z is H thus obtained into a pharmaceutically acceptable salt thereof.

**2.** A process according to claim 1 wherein the anthracycline glycoside of formula II or salt thereof dissolved in an anhydrous polar solvent is reacted, at room temperature and for 24 hours, with an excess of bis(2-iodoethyl)ether in the presence of triethylamine; the pH of the reaction mixture, previously diluted with water, is adjusted to 7.5 using an aqueous solution of sodium hydrogen carbonate; the reaction mixture is extracted with methylene chloride; the organic solvent is removed under reduced pressure; and, after purification of the crude product on a column of silica gel using a mixture of methylene chloride and methanol (98:2 v/v) as eluting agent, the product is isolated as the hydrochloride salt by addition of a methanolic solution of anhydrous hydrogen chloride.

European Patent Office

# EUROPEAN SEARCH REPORT

Application Number

EP 92 10 2929

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-4 301 277 (E. M. ACTON ET AL.)<br>* column 3, line 51 - column 4, line 15; claims 1-7 *<br>--- | 1,6 | C07H15/252<br>C07H19/02<br>A61K31/70 |
| Y | US-A-4 374 980 (H. UMEZAWA ET AL.)<br>* column 5, line 64 - column 6, line 13; claim 1 *<br>--- | 1,6 | |
| Y | EP-A-0 288 268 (FARMITALIA CARLO ERBA)<br>* page 10, line 45 - page 11, line 60; claims 1-4 *<br>----- | 1,6 | |
| | | | TECHNICAL FIELDS SEARCHED (Int. Cl.5 ) |
| | | | C07H<br>A61K |

The present search report has been drawn up for all claims

| Place of search | Date of completion of the search | Examiner |
|---|---|---|
| THE HAGUE | 20 MARCH 1992 | BRENNAN J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document